# EUROPÄISCHE PATENTSCHRIFT

(11) **EP 0 884 059 B1**
(45) Veröffentlichungstag und Bekanntmachung des Hinweises auf die Patenterteilung: **18.08.2004**
(21) Anmeldenummer: 98108761.2
(22) Anmeldetag: 14.05.1998
(51) Int. Cl.: A61M 5/14, A61M 5/20, A61M 5/142, A61M 39/24

(54) **Differenzdruck-Ventil**
Differential pressure valve
Vanne à pression différentielle

(30) Priorität: 14.05.1997 DE 19720054
(43) Veröffentlichungstag der Anmeldung: 16.12.1998
(73) Patentinhaber: Filtertek B.V., County of Limerick (IE)
(72) Erfinder: Myers, Jan Willem Marinus, 5913 TP Venlo (NL)
(74) Vertreter: Brose, D. Karl

(56) Entgegenhaltungen:
- EP-A- 0 562 246
- WO-A-89/02764
- DE-A- 4 142 494
- FR-A- 2 666 745
- US-A- 3 633 605

## Beschreibung

Die Erfindung betrifft ein Differenzdruck-Ventil zur schwallartigen Verabreichung von Flüssigmedikamenten, Kontrastmitteln oder dergleichen mit zwei Eingängen welche je eine zugeordnete Differenzenkraftkammer aufweisen, die mit einer gemeinsamen Ableitung für das Flüssigmedikament verbunden und durch eine Membranscheibe von einander getrennt sind.

Ein Dokument, des die Merkmale der Prëambel des Anspruchs 1 offenbart, ist die WO 89/02764.

Der vorliegenden Erfindung liegt die Aufgabe zu Grunde, eine neue Verwendung eines derartigen Ventil zu ermöglichen, nämlich diese zur schwallartigen Verabreichung von Flüssigmedikamenten und dergleichen auszugestalten.

Diese Aufgabe wird mit den Merkmalen des Anspruchs 1 gelöst.

Bei einer bevorzugten Ausführungsform nach der Erfindung ist der erste Eingang mit einem männlichen Luerlock-Anschluss versehen und der zweite Eingang weist einen weiblichen Luerlock-Anschluss auf.

Nach der Erfindung ist vorgesehen, dass das Ventil aus zwei dichtend miteinander verbindbaren Ventilgehäusehälften gebildet ist, wobei eine Ventilgehäusehälfte den ersten Eingang und die zweite Ventilgehäusehälfte die Ableitung und den zweiten Eingang aufweist.

Die beiden miteinander verbindbaren Ventilgehäusehälften weisen innerhalb der Differenzkraftkammern jeweils konzentrisch zu einem Flüssigkeitseinlass bzw. Flüssigkeitsauslass ringförmige Stege auf, wobei dem ersten Eingang der mit dem Behälter verbunden ist, ein Ringsteg mit größerem Durchmesser und der Ableitung ein Ringsteg mit kleinerem Durchmesser zugeordnet ist.

Bevorzugt ist es ferner hierbei, dass die Membranscheibe mit einem Teil ihres Umfangs an einer Öffnung liegt, die in den zweiten Eingang mündet.

Eine Verbesserung der Erfindung besteht noch darin, dass der koaxial zu dem Ringsteg mit größerem Durchmesser mündende Flüssigkeitsauslass der Ableitung abgewinkelt geformt ist und der koaxial zu dem anderen Ringsteg mündende Flüssigkeitseinlass des ersten Eingangs koaxial zu diesem verläuft.

Die angestrebte Ventilwirkung mit hervorragender Dichtigkeit bei sehr kleinen Drücken wird dadurch erzielt, dass die Membranscheibe eine kreisrunde Form aufweist und aus einer Flüssig-Silikon-, Silikon- oder Naturgummibahn oder einer Flüssig-Silikon-, Silikon- oder Naturgummimatte hergestellt ist.

Im folgenden wird die Erfindung anhand einer in der Zeichnungen beispielhaft veranschaulichten Ausführungsform näher erläutert. Es zeigt:
**Figur 1** eine seitliche Schnittansicht durch das Differenzdruckventil in seiner Ausführungsform als Dreiwege-Rückschlagventil;
**Figur 2** eine Unteransicht des Ventils gemäß Fig. 1 und
**Figur 3** das Ventil in natürlicher Größe.

Bei der Darstellung in den Zeichnungen wurden die weiteren Einzelheiten, nämlich der Behälter für Flüssigkeitsmedikamente, und die Injektionsspritze bzw.pumpe, da diese dem Fachmann bekannt sind und da das Differenzdruckventil Kernstück der vorliegenden Erfindung bildet.

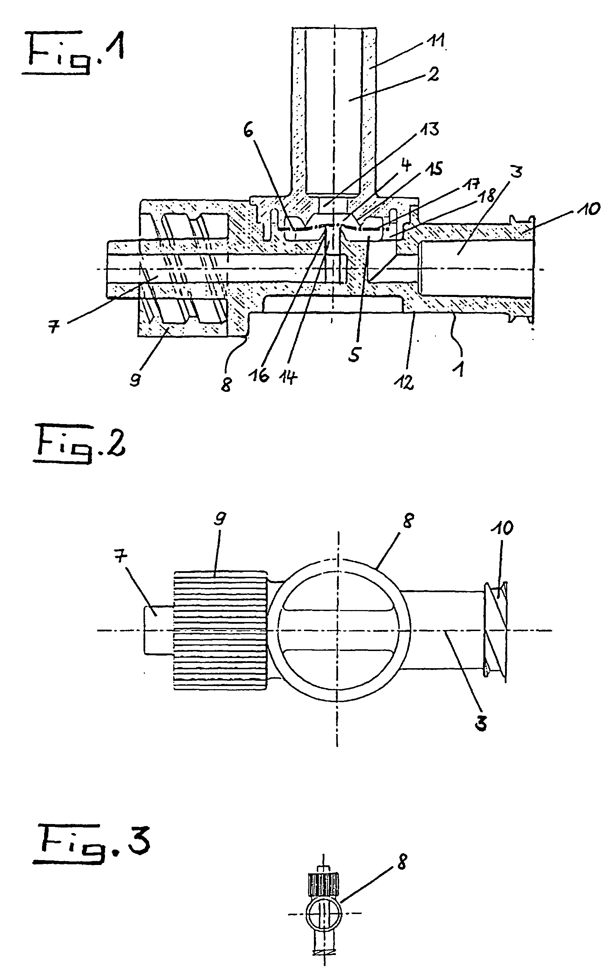

Wie insbesondere aus Fig. 1 ersichtlich, weist das Differenzdruckventil 1 zwei Eingänge 2 und 3 auf, die je in eine Differenzkraftkammer 4 bzw. 5 münden. Die beiden Differenzkraftkammern 4 und 5 sind durch eine Membranscheibe 6 voneinander abgedichtet getrennt. Beide Differenzkraftkammern 4 und 5 stehen gemeinsam mit einer Ableitung 7 für das Flüssigmedikament in Verbindung, wobei an die Ableitung 7 üblicherweise die zum Patienten führende Leitung angeschlossen ist, während der erstes Eingang 2 an die Flasche oder Behälter mit dem Flüssigmedikament über eine geeignete Leitung angeschlossen ist.

Das Differenzdruckventil 1 gemäß Fig. 1 ist als 3-Wege-Rückschlagventil 8 ausgebildet, da der erste Eingang 2 an den nicht dargestellten Behälter für das Flüssigmedikament angeschlossen ist und der zweite Eingang 3 für den Anschluss an eine ebenfalls nicht dargestellte Injektionsspritze oder dergleichen ausgebildet ist.

Wie gezeigt, ist die Ableitung 7 mit einem männlichen Luerlock-Anschluss 9 versehen, und der zweite Eingang 3 weist einen weiblichen Luerlock-Anschluss 10 auf, wobei die Abmessungen derart getroffen sind, dass durch die leichte Konizität des zweiten Eingangs 3 das Vorderende einer handelsüblichen Injektionsspritze formschlüssig aufgenommen werden kann.

Das Gehäuse des Differenzdruckventils 1 besteht aus zwei dichtend miteinander verbindbaren Ventilgehäusehälften 11 und 12. Die Ventilgehäusehälfte 11 enthält den ersten Eingang 2 und die zweite Ventilgehäusehälfte 12 enthält den zweiten Eingang 3 und die Ableitung 7. Innerhalb der Differenzkraftkammern 4 weist die Ventilgehäusehälfte 11 konzentrisch zu einem Flüssigkeitseinlass 13 einen ringförmigen Steg 15 auf. Die Ventilgehäusehälfte 2 weist innerhalb der Differenzkraftkammer 5 konzentrisch zu einem Flüssigkeitsauslass 14 einen ringförmigen Steg 16 auf, wobei die Abmessungen derart getroffen sind, dass der Ringsteg 15 mit dem größeren Durchmesser den ersten Eingang 2 zugeordnet ist, welcher mit dem Behälter für das Flüssigmedikament verbunden ist, und der Ringsteg 16 mit dem kleineren Durchmesser der Ableitung 7 die zur Tropfkammer führt, zugeordnet ist.

Die zwischen den Ventilgehäuse 11 und 12 angeordnete Membranscheibe liegt mit einem Teil 17 ihres Umfangs an einer Öffnung 18, die in den zweiten Eingang 3 mündet. Der koaxial zu dem Ringsteg 16 mit kleinerem Durchmesser mündende Flüssigkeitsauslass 14 der Ableitung 7 ist wie aus Fig-. 1 ersichtlich abgewinkelt geformt, während der koaxial zu dem Ringsteg 15 mündende Flüssigkeitseinlass 13 des Eingangs 2 koaxial zu diesem Eingang liegt.

Die kreisrunde Membranscheibe 6 ist aus einer Flüssig-Silikon-, Silikon- oder Naturgummibahn, oder einer Flüssig-Silikon-, Silikon- oder Naturgummimatte hergestellt.

Die Funktionsweise des 3-Wege-Rückschlagventils 8 ist derart, dass mittels einer Injektionsspritze oder -pumpe am Eingang 3 aus dem Flüssigmedikamentsbehälter Flüssigkeit angesaugt wird, so dass kurzzeitig die Membran 6 gegen den Ringsteg 16 aufgrund des auf der Unterseite erzeugten Unterdrucks gedrückt wird. Hierbei wird nicht aus der zum Patienten führenden Leitung Flüssigkeit entnommen. Wird nun zusätzliche Flüssigkeit oder ggf. ein anderes Medikament am zweiten Eingang 3 injiziert, so wird aufgrund des sich entsprechend aufbauenden Überdrucks die Membran 6 gegen den oberen Ringsteg 15 gedrückt, so dass die zusätzliche Flüssigkeit ausschließlich von dem zweiten Eingang 3 zum Patienten über die Ableitung 7 gelangt und nicht in die zum Behälter zurückführende Leitung am ersten Eingang 2 gelangen kann.

Es ist offensichtlich, dass auf diese Weise ein ausgesprochen kompaktes 3-Wege-Rückschlagventil geschaffen wurde, welches lediglich aus drei Einzelteilen besteht, nämlich der ersten Ventilgehäusehälfte 11, der zweiten Ventilgehäusehälfte 12 und der dazwischenliegenden Membran 6.

Sämtliche der aus der Beschreibung, den Ansprüchen und Zeichnungen hervorgehenden Merkmale und Vorteile der Erfindung, einschließlich konstruktiver Einzelheiten und räumlicher Anordnungen, können sowohl für sich als auch in beliebiger Kombination erfindungswesentlich sein.

### BEZUGSZEICHENLISTE

- 1 =: Differenzdruckventil
- 2 =: Erster Eingang
- 3 =: Zweiter Eingang
- 4 =: Differenzkraftkammer
- 5 =: Differenzkraftkammer
- 6 =: Membranscheibe
- 7 =: Ableitung
- 8 =: 3-Wege-Rückschlagventil
- 9 =: männlicher Luerlock-Anschluss
- 10: = weiblicher Luerlock-Anschluss
- 11 =: Ventilgehäusehälfte
- 12 =: Ventilgehäusehälfte
- 13 =: Flüssigkeitseinlass
- 14 =: Flüssigkeitsauslass
- 15 =: Ringsteg
- 16 =: Ringsteg
- 17 =: Teil des Umfangs von 6
- 18 =: Öffnung

## Patentansprüche

1. Differenzdruck-Ventil zur schwallartigen Verabreichung von Flüssigmedikamenten, Kontrastmitteln und der Gleichen mit zwei Eingängen welche je eine zugeordnete Differenzenkraftkammer aufweisen welche mit einer gemeinsamen Ableitung für das Flüssigmedikament verbunden und durch eine Membranscheibe von einander getrennt sind, wobei das Differenzdruck-Ventil (1), das aus zwei dichtend miteinander verbindbaren Ventilgehäusehälften (11, 12) gebildet ist, wobei eine Ventilgehäusehälfte (11) den ersten Eingang (2) und die zweite Ventilgehäusehälfte (12) die Ableitung (7) und den zweiten Eingang (3) aufweist, als Dreiwegerückschlagventil (8) ausgebildet ist indem der erste Eingang (2) an den Behälter für das Flüssigkeitsmedikament Kontrastmittel oder der Gleichen anschliessbar ist und der zweite Eingang für den Anschluss an eine Injektionsspritze oder der Gleichen ausgebildet ist, derart, dass bei Unterdruck am zweiten Eingang (3) die Ableitung (7) und bei Überdruck am zweiten Eingang (3) der erste Eingang (2) geschlossen ist, und dass bei Unterdruck am zweiten Eingang (3) der erste Eingang (2) und der zweite Eingang (3) und bei Überdruck am zweiten Eingang (3) der zweite Eingang (3) und die Ableitung (7) verbunden sind, **dadurch gekennzeichnet, dass** die beiden miteinander verbindbaren Ventilgehäusehälften (11, 12) innerhalb der Differenzkraftkammern (4, 5) jeweils konzentrisch zu einem Flüssigkeitseinlass (13) bzw. Flüssigkeitsauslass (14) ringförmige Stege (15, 16) aufweisen, wobei dem ersten Eingang (2) der mit dem Behälter verbindbar ist, ein Ringsteg (15) mit größerem Durchmesser und der Ableitung (7) ein Ringsteg (16) mit kleinerem Durchmesser zugeordnet ist.

2. Ventil nach Anspruch 1, **dadurch gekennzeichnet, dass** die Ableitung (7) mit einem männlichen Luerlock-Anschluß (9) versehen ist und der zweite Eingang (3) einen weiblichen Luerlock-Anschluß (10) aufweist.

3. Ventil nach Anspruch 1 oder 2, **dadurch gekennzeichnet, dass** die Membranscheibe (6) mit einem Teil (17) ihres Umfangs an einer Öffnung (18) liegt, die in den zweiten Eingang (3) mündet.

4. Ventil nach Anspruch 1 oder 3, **dadurch gekennzeichnet, dass** der koaxial zu dem Ringsteg (16) mit kleinerem Durchmesser mündende Flüssigkeitsauslass (14) der Ableitung (7) abgewinkelt geformt ist, und dass der koaxial zu dem Ringsteg (15) mündende Flüssigkeitseinlass (13) des ersten Eingangs (2) koaxial zu diesem verläuft.

5. Ventil nach einem der Ansprüche 1 bis 4, **dadurch gekennzeichnet, dass** die Membranscheibe (6) eine kreisrunde Form aufweist und aus einer Flüssig-Silikon-, Silikon- oder Naturgummi-Bahn oder einer Flüssig-Silikon-, Silikon- oder Naturgummimatte hergestellt ist.

## Claims

1. Differential pressure valve for the surge-like administration of liquid medications, contrast agents and the like, having two inlets which each are having an associated differential force chamber which are connected to a common exit line for the liquid medication and which are separated from each other by a diaphragm disk, wherein the differential pressure valve 1 which is formed of two valve housing halves (11, 12) being sealingly connectable with each other, wherein one valve housing half (11) is containing the first inlet and the second valve housing half (12) is containing the exit line (7) and the second inlet (3), is performed as a three-way check valve (8) by the fact that the first inlet (2) is connected to the container for the liquid medication and the second inlet (3) is performed for being connected to a syringe or the like such that with negative pressure at the second inlet (3) the exit line (7) is closed and with overpressure at the second inlet (3) the first inlet (2) is closed and that with negative pressure at the second inlet (3) the first inlet (2) and the second inlet (3) and with overpressure at the second inlet (3) the second inlet (3) and the exit line (7) are connected, **characterized in that** the two housing halves (11, 12) being connectable with each other are having circular rims (15, 16) within the differential force chambers (4, 5) each concentric to a fluid inlet (13) or a fluid outlet (14), respectively, wherein a circular rim (15) having a larger diameter is associated with the first inlet (2) being connectable to the container and a second circular rim (16) having a smaller diameter being associated with the exit line (7).

2. Valve according to claim 1, **characterized in that** the exit line (7) is provided with a male Luerlock-connector (9) and that the second inlet (3) is having a female Luerlock-connector (10).

3. Valve according to claim 1 or 2, **characterized in that** the diaphragm disk (6) with a part (17) of its circumference is positioned adjacent to an opening (18) leading into the second inlet (3).

4. Valve according to claim 1 or 3, **characterized in that** the fluid outlet (14) of the exit line (7) which is opening coaxially to the circular rim (16) having the smaller diameter is having an angular shape and that the fluid inlet (13) which is opening coaxially to the circular rim (15) is extending coaxially to the first inlet (2).

5. Valve according to any of the claims 1 to 4, **characterized in that** the diaphragm disk (6) is having a circular shape and is produced from a fluid silicon-, silicon- or natural rubber sheet or from a fluid silicon-, silicon- or natural rubber mat.

## Revendications

1. Soupape à pression différentielle, pour administrer par vagues des médicaments liquides, des agents de contraste et analogues, comportant deux entrées avec chacune une chambre à force différentielle associée, reliées ensemble à une conduite d'évacuation pour le médicament liquide et séparées l'une de l'autre par un disque formant membrane, la soupape à pression différentielle (1)qui est constituée de deux moitiés de boîtier de soupape (11, 12) pouvant être reliées entre elles de manière étanche, une moitié de boîtier de soupape (11) présentant la première entrée (2) et la deuxième moitié de boîtier de soupape (12) présentant la conduite d'évacuation (7) et la deuxième entrée (3), étant réalisée en en tant que clapet de non-retour à trois voies (8), en ce que la première entrée (2) est raccordée au récipient du médicament liquide, d'agent de contraste ou analogue et la deuxième entrée (3) est réalisée pour le raccord à une seringue de perfusion ou similaire, de manière qu'en cas de dépression à la deuxième entrée (3), la conduite d'évacuation (7) et en cas de surpression à la deuxième entrée (3), la première entrée (2) soient fermées, et en ce que en cas de dépression à la deuxième entrée (3) la première entrée (2) et la deuxième entrée (3) soient raccordées et en cas de surpression à la deuxième entrée (3) , la deuxième entrée (3) et la conduite d'évacuation (7 soient raccordées,
**caractérisé en ce que** les deux moitiés de boîtier de soupape (11, 12) pouvant être reliées entre elles présentent, à l'intérieur des chambres à force différentielle (4, 5), des nervures annulaires (15, 16) concentriques à une entrée de liquide (13) ou une sortie de liquide (14), une nervure annulaire (15) de plus grand diamètre étant associée à la première entrée (2), qui est reliée au récipient, et une nervure annulaire (16) de plus petit diamètre étant associée à la conduite d'évacuation (7).

2. Soupape selon la revendication 1, **caractérisé en ce que** la conduite d'évacuation (7) est pourvue d'un raccord Luerlock mâle (9) et la deuxième entrée (3) d'un raccord Luerlock femelle (10).

3. Soupape selon l'une des revendications 1 ou 3, **caractérisé en ce que** le disque à membrane (6) se trouve, par une partie (17) de sa périphérie, sur une ouverture (18) qui débouche dans la deuxième entrée (3).

4. Soupape selon la revendication 1 ou 3, **caractérisé en ce que** la sortie de liquide (14) de la conduite d'évacuation (7) débouchant coaxialement à la nervure annuaire (16) de plus petit diamètre, est coudée et **en ce que** l'entrée de liquide (13) débouchant coaxialement à la nervure annulaire (15) de la première entrée (2) s'étend coaxialement à celle-ci.

5. Soupape selon l'une des revendications 1 à 4, **caractérisé en ce que** le disque à membrane (6) présente une forme circulaire et est réalisé dans une bande de silicone liquide, de caoutchouc au silicone, de caoutchouc naturel ou dans un mat de silicone liquide, de caoutchouc au silicone ou de caoutchouc naturel.
